(19) Europäisches Patentamt
European Patent Office
Office européen des brevets

(11) Numéro de publication: **0 342 105**
**A1**

(12) **DEMANDE DE BREVET EUROPEEN**

(21) Numéro de dépôt: 89401259.0

(22) Date de dépôt: 03.05.89

(51) Int. Cl.4: **C 07 D 275/02**
C 07 D 275/04,
C 07 D 513/00, A 61 K 31/41,
A 61 K 31/435, A 61 K 7/00,
A 61 K 7/06, A 61 K 7/48,
C 07 C 149/00,
C 07 D 213/81, C 09 D 3/14

(30) Priorité: 09.05.88 FR 8806222

(43) Date de publication de la demande:
15.11.89 Bulletin 89/46

(84) Etats contractants désignés:
BE CH DE FR GB IT LI NL SE

(71) Demandeur: CENTRE INTERNATIONAL DE RECHERCHES DERMATOLOGIQUES C.I.R.D.
Groupement d'Intérêt Economique dit:
Sophia Antipolis
F-06560 Valbonne (FR)

(72) Inventeur: Shroot, Braham
Villa 35 Hameaux de Val Bosquet
Chemin de Val Bosquet F-06600 Antibes (FR)

Maignan, Jean
8, rue Halévy
F-93290 Tremblay Les Gonesse (FR)

Schmidt, Rainer
22, Place des Arcades
F-06250 Mougins (FR)

(74) Mandataire: Stalla-Bourdillon, Bernard et al
CABINET NONY & CIE 29, rue Cambacérès
F-75008 Paris (FR)

(54) **Adamantyl-2 isothiazoline-4 ones-3, leur procédé de préparation et leur utilisation comme agents bactéricides et fongicides.**

(57) Adamantyl-2 isothiazoline-4 ones-3, correspondant à la formule générale suivante :

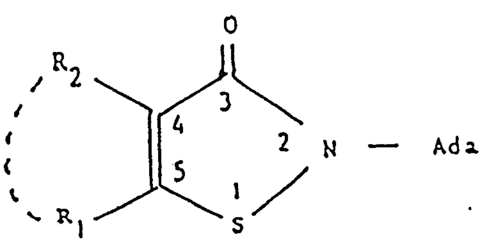

(I)

dans laquelle
$R_1$ et $R_2$ , identiques ou différents, représentent un atome d'hydrogène, ou un atome d'halogène,
ou $R_1$ et $R_2$ , pris ensemble, forment un chaîne polyméthylène ayant 3 ou 4 atomes de carbone éventuellement substituée par un radical alkyle inférieur ou par un halogène,
ou encore $R_1$ et $R_2$ , pris ensemble, forment un cycle pyridinique avec la double liaison du noyau isothiazoline-4 one-3,
et Ada représente un radical adamantyle pris dans le groupe constitué par Adamantyl-1′ , Adamantyl-2′ et Adamantyl-1′ méthyl, et leurs sels d'un acide minéral ou organique.

Ces composés trouvent une application comme agents bactéricides et fongicides dans divers domaines de l'industrie.

EP 0 342 105 A1

## Description

### Adamantyl-2 isothiazoline-4 ones-3, leur procédé de préparation et leur utilisation comme agents bactéricides et fongicides.

La présente invention a pour objet de nouveaux dérivés d'isothiazoline-4 one-3, plus particulièrement des adamantyl-2 isothiazoline-4 ones-3, leur procédé de préparation et leur utilisation comme agents bactéricides et fongicides.

Dans le brevet français n°80.22278 (2.492.376), il a déjà été proposé des polyméthylène-4,5 isothiazoline-4 ones-3 de formule générale:

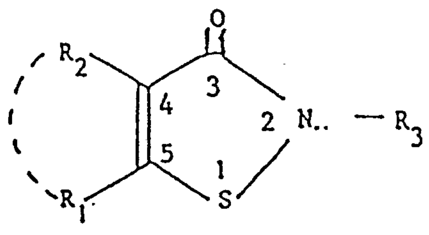

dans laquelle:

$R_1$ et $R_2$ pris ensemble, représentent une chaîne polyméthylène ayant de 3 à 4 atomes de carbone ou une chaîne polyméthylène ayant de 3 à 4 atomes de carbone substituée par un radical Alkyle inférieur ayant de 1 à 4 atomes carbone,

et $R_3$ étant susceptible de représenter différents radicaux, notamment un radical cycloalkyle ayant de 3 à 6 atomes de carbone.

Les études effectuées sur ces isothiazoline-4 ones-3 substituées en position 2 par un radical cycloalkyle ont permis de mettre en évidence que ces composés étaient effectivement actifs à l'égard des bactéries, mais que leur activité était toutefois inférieure à celle des dérivés substitués en position 2 par un radical alkyle, linéaire ou ramifié, ayant de 1 à 4 atomes de carbone.

A la suite de nouvelles études, on vient maintenant de constater de façon tout à fait surprenante, que les isothiazoline-4 ones-3 substituées en position 2 par un radical adamantyle présentaient une excellente activité bactéricide et fongicide et qu'elles étaient moins toxiques chez les mammifères.

On a en particulier noté que ces nouveaux dérivés substitués en position 2 par un radical adamantyle étaient des antifongiques particulièrement puissants.

La présente invention a donc pour objet des adamantyl-2 isothiazoline-4 ones-3 qui peuvent être représentées par la formule générale suivante:

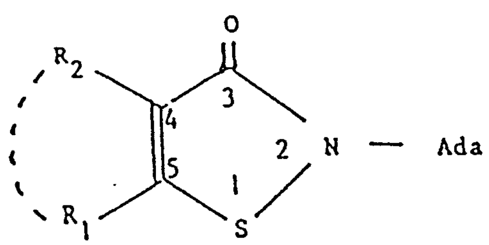

(I)

dans laquelle:

$R_1$ et $R_2$ , identiques ou différents, représentent un atome d'hydrogène, ou un atome d'halogène,

ou $R_1$ et $R_2$ , pris ensemble, forment une chaîne polyméthylène ayant 3 ou 4 atomes de carbone éventuellement substituée par un radical alkyle inférieur ou par un halogène,

ou encore $R_1$ et $R_2$ pris ensemble forment un cycle pyridinique avec la double liaison du noyau isothiazoline-4 one-3,

et Ada représente un radical adamantyle pris dans le groupe constitué par:

(i) 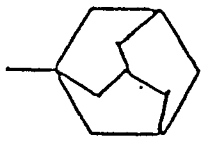 (Adamantyl-1')

(ii) 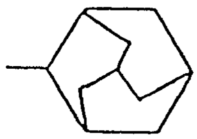 (Adamantyl-2')

(iii) 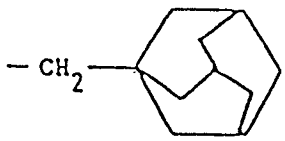 (Adamantyl-1' méthyl)

et leurs sels d'un acide minéral ou organique.

L'atome d'halogène des radicaux $R_1$ et $R_2$ est de préférence le fluor, le brome ou le chlore.

Lorsque les radicaux $R_1$ et $R_2$ , pris ensemble, forment un cycle pyridinique avec la double liaison du noyau isothiazoline-4 one-3, celui-ci peut être substitué par un ou plusieurs alkyle(s) inférieur(s) ayant de 1 à 4 atomes de carbone ou par au moins un atome d'halogène tel que le fluor, le chlore ou le brome ou encore par un groupement nitro.

Selon une première forme de réalisation de l'invention, les adamantyl-2 isothiazoline-4 ones-3 peuvent être représentées par la formule générale:

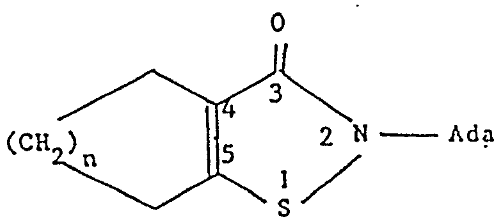 (II)

dans laquelle:
Ada à la même signification que celle donnée pour la formule (I)
et n est 1 ou 2.

Il s'agit alors d'adamantyl-2 triméthylène ou tétraméthylène-4,5 isothiazoline-4 ones-3.

Selon une deuxième forme de réalisation de l'invention, les adamantyl-2 isothiazoline-4 ones-3 peuvent être représentées par la formule générale suivante:

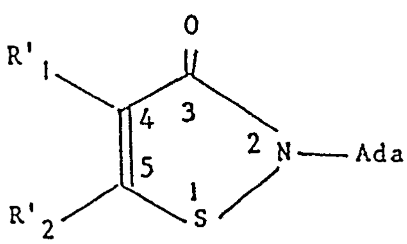 (III)

dans laquelle:
Ada a la même signification que celle donnée pour la formule (I),
et $R'_1$ et $R'_2$ ,identiques ou différents, représentent un atome d'hydrogène ou un atome d'halogène tel qu'un atome de fluor, de chlore ou de brome.

Selon cette forme de réalisation, le radical Ada est de préférence le radical adamantyl-1' ou adamantyl-2'.

Enfin, selon une troisième forme de réalisation, les adamantyl-2 isothiazoline-4 ones-3 peuvent être représentées par la formule générale suivante:

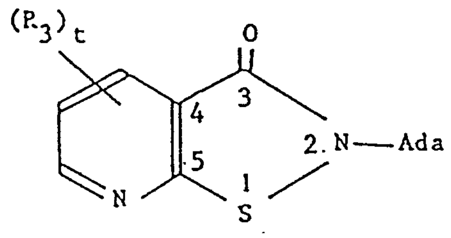

(IV)

dans laquelle:
Ada a la même signification que celle donnée pour la formule (I),
et $R_3$ représente un atome d'hydrogène, un radical alkyle inférieur ayant de 1 à 4 atomes de carbone, un atome de fluor, de chlore ou de brome ou un groupement nitro,
et t est 1 ou 2.

Il s'agit alors d'adamantyl-2 isothiazolo-(5,4b) pyridinones-3 éventuellement substituée(s).

Lorsque les composés selon l'invention se présentent sous forme de sels d'un acide minéral ou organique il s'agit plus particulièrement de chlorhydrates, bromhydrates, nitrates, sulfates ou succinates.

Les adamantyl-2 isothiazoline-4 ones-3 particulièrement préférées selon l'invention sont celles représentées par la formule générale (III) dans laquelle le radical Ada représente un radical adamantyl-1′ ou adamantyl-2′ et $R'_1$ et $R'_2$ représentent un atome d'hydrogène ou un atome de chlore, l'un au moins des radicaux $R'_1$ ou $R'_2$ représentant un atome de chlore.

Parmi les composés selon l'invention qui correspondent aux formules (I) à (IV) on peut en particulier citer les suivants:

- (Adamantyl-1′)-2-triméthylène-4,5 isothiazoline-4 one-3,
- (Adamantyl-1′ méthyl)-2-triméthylène-4,5 isothiazoline-4 one-3,
- (Adamantyl-2′)-2-triméthylène-4,5 isothiazoline-4 one-3,
- (Adamantyl-2′)-2 isothiazolo-(5,4b) pyridinone-3,
- (Adamantyl-2′)-2 isothiazoline-4 one-3,
- (Adamantyl-2′)-2 chloro-5 isothiazoline-4 one-3,
- (Adamantyl-2′)-2 dichloro-4,5 isothiazoline-4 one-3,
- (Adamantyl-1′)-2 isothiazoline-4 one-3,
- (Adamantyl-1′)-2 chloro-5 isothiazoline-4 one-3,
- (Adamantyl-1′)-2 dichloro-4,5 isothiazoline-4 one-3,
- (Adamantyl-1′)-2 bromo-5 isothiazoline-4 one-3,
- (Adamantyl-1′)-2 bromo-4 chloro-5 isothiazoline-4 one-3,
- (Adamantyl-2′)-2 bromo-4 chloro-5 isothiazoline-4 one-3.

Les composés selon l'invention peuvent être préparés par divers procédés, notamment par le procédé décrit par GOERDELER et MITTLER, Chem. Ber. 96, 944-954 (1963) qui consiste à cycliser en milieu solvant organique inerte une carbamoyl-2 thiocyclanone (1) selon le schéma réactionnel suivant:

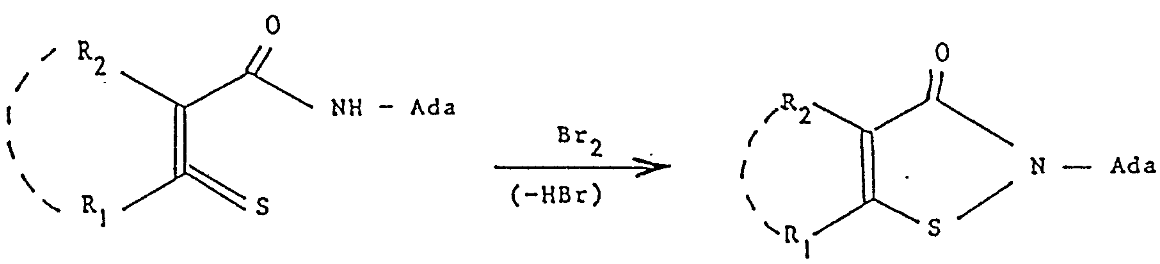


(1)

Pour la préparation des adamantyl-2 polyméthylène-4,5 isothiazoline-4 ones-3, on préfère toutefois utiliser le procédé décrit dans le brevet français n°85.08469 (2.583.046).

Ce procédé peut être également avantageusement utilisé pour la préparation des adamantyl-2 isothiazolo-(5,4b) pyridinones-3.

Ce procédé peut être représenté par le schéma réactionnel suivant:

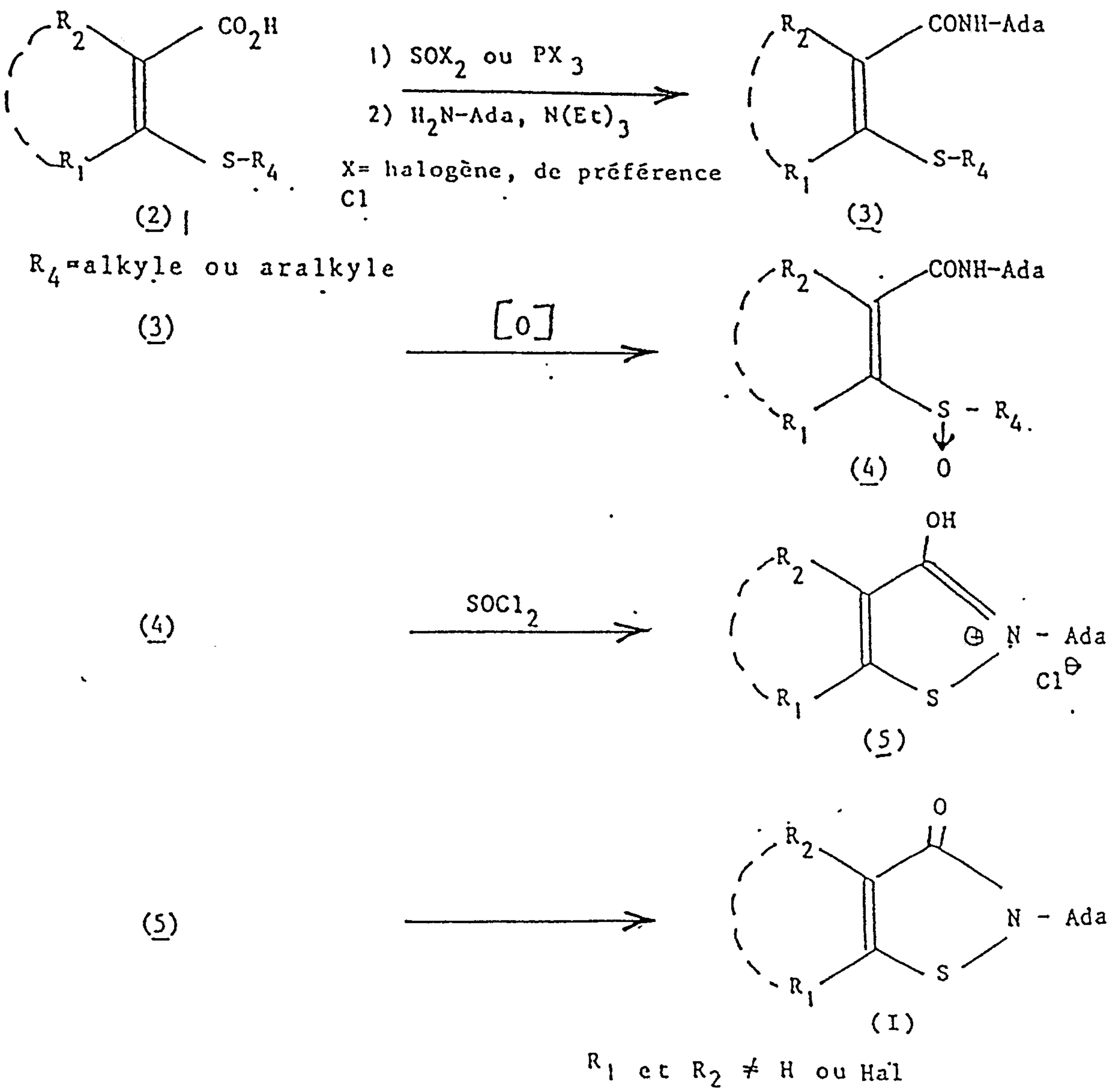


(2) (3)

$R_4$=alkyle ou aralkyle

(3) (4)

(4) (5)

(5) (I)

$R_1$ et $R_2$ ≠ H ou Hal

La première étape de ce procédé consiste à préparer un alkyl ou aralkyl thio-2 carboxamide (3) à partir d'un acide alkyl ou aralkyl thio-2 carboxylique (2) en le transformant en chlorure d'acide par traitement avec le chlorure de thionyle ou un trihalogénure de phosphore (de préférence le trichlorure de phosphore) dans un solvant aromatique tel que le toluène ou un solvant chloré tel que le dichloro-1,2 éthane ou le chlorure de méthylène. Ce chlorure d'acide intermédiaire est ensuite transformé en alkyl ou aralkyl thio-2 carboxamide (3) en le faisant réagir sur une adamantylamine($H_2N$-Ada) en présence de triéthylamine.

Les sulfoxides (4) des composés de formule (3) sont obtenus par action soit de l'acide m-chloroperbenzoïque soit par action d'un peracide organique formé in situ par addition d'eau oxygénée sur un acide organique tel que l'acide formique ou l'acide acétique. Cette réaction est réalisée en solubilisant l'alkyl ou aralkyl thio-2 carboxamide (3) dans cet acide organique pur ou en mélange avec un solvant chloré tel que le chlorure de méthylène, puis on ajoute alors goutte à goutte à 0° C l'eau oxygénée. Après la fin de l'addition le mélange est ensuite agité de 1 à 2 heures à température ambiante et on obtient avec un très bon rendement le dérivé sulfoxyde (4) correspondant que l'on purifier selon les méthodes conventionnelles.

La réaction de cyclisation des sulfoxydes (4) en vue d'obtenur les chlorhydrates des adamantyl-2 isothiazoline-4 ones-3 de formule (5) est et préférence conduite dans un solvant organique tel que par exemple le chlorure de méthylène en présence d'un chlorure d'acide tel que le chlorure de thionyle.

Cette réaction de cyclisation est très sélective et les chlorhydrates attendus précipitent au sein du mélange réactionnel; toutefois lorsqu'ils sont solubles ils peuvent être facilement précipités par addition d'éther isopropylique.

Les chlorhydrates des adamantyl-2 isothiazoline-4 ones-3 (5) étant particulièrement lipophiles, il est très aisé de les transformer en base libre par simple lavage à l'eau d'une solution ou d'une suspension du chlorhydrate dans un solvant organique tel que le toluène, le dichlorométhane ou encore le chlorure de

méthylène. En général, après le troisième lavage à l'eau, la phase aqueuse ne contient plus d'ion chlorure.

Les produits de départ de ce procédé de préparation des composés selon l'invention sont (i) les acides polyméthylène alkyl ou aralkyl thio-2 carboxyliques et (ii) les acides alkyl ou aralkyl thio-2 nicotiniques.

La préparation des acides polyméthylène alkyl ou aralkyl thio-2 carboxyliques est décrite dans le brevet français n°85.08469 (2.583.046).

Les acides alkyl ou aralkyl thio-2 nicotiniques sont obtenus à partir de l'acide chloro-2 nicotinique par réaction de déplacement du chlore par un thiolate, de potassium ou de sodium, préparé à partir d'un alkyl ou aralkyl mercaptan de formule $R_4$ SH.

Les composés selon l'invention de formule générale (I) dans laquelle $R_1$ et/ou $R_2$ représentent un atome d'hydrogène ou un atome d'halogène, sont de préférence obtenus au départ de dérivés de mercapto-3 propionamide selon la méthode décrite par S. N. LEWIS et al, J. Heter Chem. 571, 1971.

Ce procédé peut être représenté par le schéma réactionnel suivant:

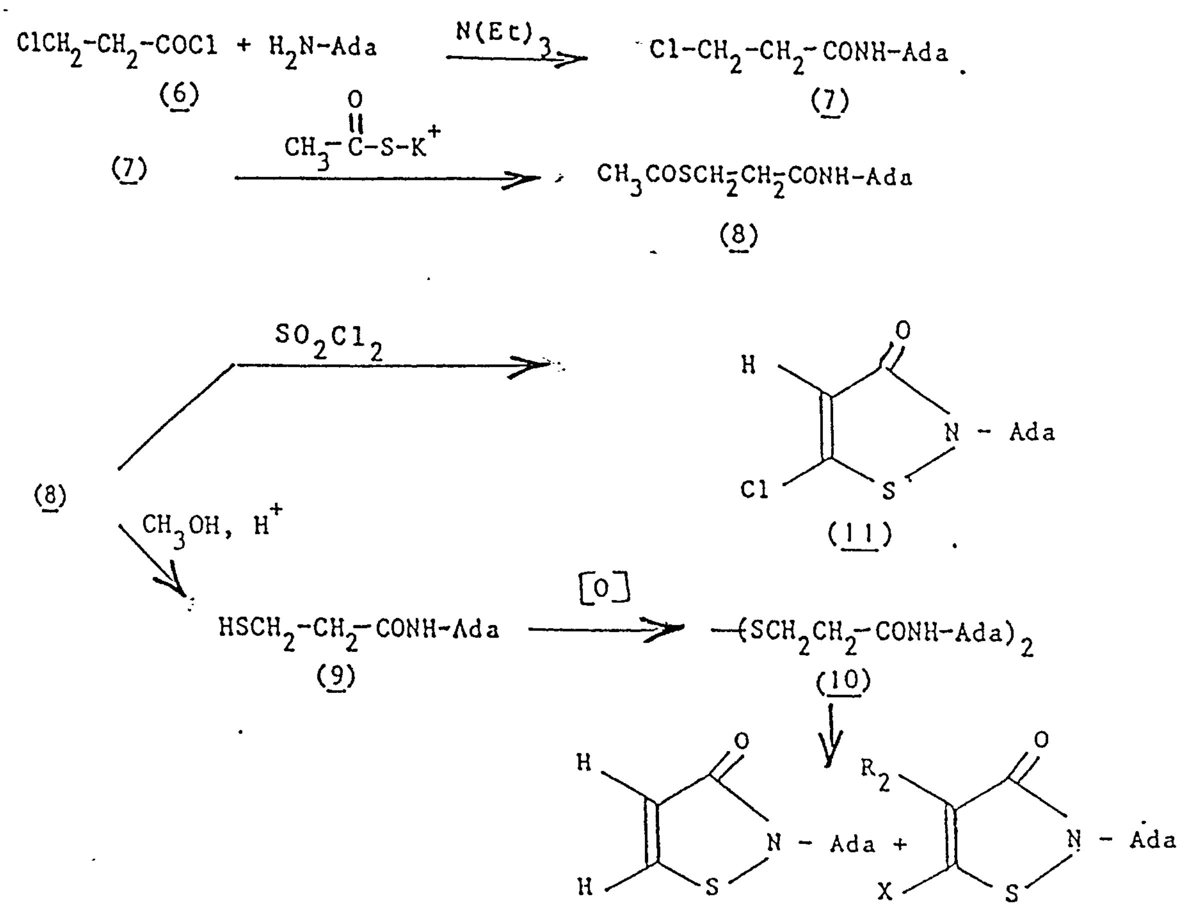


(6) (7) (8) (9) (10) (11)

$R_2$ = H ou X

Le N-adamantyl chloro-3 propionamide (7) est obtenu en faisant réagir le chlorure de l'acide chloro-3 propionique sur une adamantylamine ($H_2N$-Ada), en présence de triéthylamine.

Cet amide (7) est mis alors à réagir avec le thiolacétate de potassium dans un solvant organique tel que l'acétone et conduit avec un bon rendement au N-adamantyl acétylthio-3 propionamide (8). A partir de ce dernier, il est possible d'accéder aux adamantyl-2 isothiazoline-4 ones-3 selon deux voies de synthèse.

Selon la première voie, le N-adamantyl acétylthio-3 propionamide (8) est transformé en thiol correspondant (9) par traitement dans le méthanol en milieu acide. Ce dernier est alors oxydé en présence d'eau oxygénée en disulfure (10) correspondant. Par réaction avec un halogène, le disulfure (10) est transformé en adamantyl-2 isothiazoline-4 one-3. Suivant les quantités d'halogène utilisées, on peut orienter la formation soit d'isothiazoline-4 one-3 non-halogénée et mono-halogénée soit de mono et dihalogéno-4,5 isothiazoline-4 ones-3.

Selon la deuxième voie on fait réagir le chlorure de sulfuryle directement sur le N-adamantyl acétylthio-3 propionamide (8) solubilisé dans le toluène. On obtient ainsi comme produit prépondérant l'adamantyl-2 chloro-5 isothiazoline-4 one-3 (11).

L'invention a également pour objet cette deuxième voie de synthèse des adamantyl-2 isothiazoline-4 ones-3 mono-halogénées en position 5, celle-ci étant particulièrement avantageuse car elle évite la préparation du

thiol (9) et de son disulfure (10) et de plus la mono-halogénation de la double liaison est plus sélective.

L'invention a en outre pour objet à titre de produits industriels nouveaux les composés intermédiaires de synthèse de formule suivante:

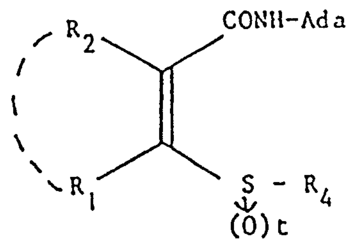

(V)

dans laquelle:
t est 0 ou 1,
Ada a la même signification que celle donnée pour la formule (I)
$R_1$ et $R_2$ ont les mêmes significations que celles données pour la formule (I) à l'exclusion de $R_1$ et $R_2$ , identiques ou différents, représentant un atome d'hydrogène ou un atome d'halogène, et
$R_4$ représente un radical alkyle ou aralkyle.

Comme ceci a été indiqué précédemment, les adamantyl-2 isothiazoline-4 ones-3 selon l'invention sont particulièrement utiles dans la destruction des bactéries, des champignons, des moisissures, des algues ainsi que de tous autres parasites.

L'invention a donc également pour objet une composition contenant de 0,01 à 25% en poids d'au moins une adamantyl-2 isothiazoline-4 one-3 telle que définie ci-dessus ou d'un sel d'addition de celle-ci en tant que composé actif. Les adamantyl-2 isothiazoline-4 ones-3 peuvent être formulées telles quelles ou sous forme d'un complexe avec la cyclodextrine dans un véhicule solide, semi-solide ou liquide.

Parmi les substances solides qui peuvent être utilisées comme véhicule approprié pour la préparation d'une composition sous la forme de poudre, on peut mentionner divers agents inertes poreux et pulvérulents de nature organique ou inorganique tels que le phosphate tricalcique, le carbonate de calcium, le kaolin, la bentonite, le talc, le Kieselguhr, l'acide borique, le liège en poudre, la sciure de bois et d'autres matières fines pulvérulentes d'origine végétale.

Le composé actif est mélangé avec les substances utilisées comme véhicule, par exemple en étant broyé avec elles. Dans une variante, la substance inerte utilisée comme véhicule peut être imprégnée avec une solution du composé actif dans un solvant facilement volatil et le solvant est ensuite éliminé par chauffage ou par filtration avec aspiration sous pression réduite. En ajoutant des agents mouillants et/ou de dispersion, aux préparations pulvérulentes qui peuvent être facilement humidifiées avec de l'eau, on peut obtenir des suspensions.

solvants inertes utilisés pour la préparation de compositions liquides doivent être non-inflammables et également inodores et non-toxiques vis-à-vis d'animaux à sang chaud ou des plantes.

Il est également possible d'utiliser des mélanges de solvants. D'autres formes liquides pouvant être utilisées, consistent en des émulsions ou des suspensions, d'un composé actif selon l'invention, dans l'eau ou des solvants inertes convenables ou également de concentrés. A cette fin, le composé actif est, par exemple, mélangé avec un agent de dispersion ou d'émulsification. Le composé actif peut également être dissous ou dispersé dans un solvant inerte approprié et mélangé simultanément ou subséquemment avec un agent de dispersion ou d'émulsification. Il est également possible d'utiliser des véhicules semi-solides et en particulier une pommade crèmeuse ou une pâte dans lesquelles le composé actif est incorporé.

En outre, il est également possible d'utiliser les composés actifs selon l'invention sous la forme d'aérosol. Le constituant actif est dans ce cas dissous ou dispersé, à l'aide de solvants inertes appropriés. On obtient des solutions sous pression qui lorsqu'elles sont pulvérisées donnent des aérosols qui conviennent particulièrement bien pour combattre les micro-organismes.

Les compositions mentionnées plus haut peuvent être appliquées par des procédés classiques tels que par saupoudrage, aspersion, pulvérisation, brossage, plongeage, enduction, imprégnation, absorption, injection ou tout autre moyen aproprié.

Les adamantyl-2 isothiazoline-4 ones-3 selon l'invention ou leurs sels peuvent être utilisées en cosmétique et entrer par exemple à titre de conservateurs dans des compositions telles que des shampooings, lotions capillaires, déodorants, produits solaires, produits pour le visage ou le corps.

Les adamantyl-2 isothiazoline-4 ones-3 peuvent être également utilisées en tant que composés actifs dans des compositions cosmétiques se présentant sous forme de solutions, dispersions, émulsions, crèmes, gels, pâtes, aérosols, poudres ou de savons.

L'invention se rapporte également aux compositions pharmaceutiques pour la médecine humaine ou vétérinaire. Ces compositions peuvent se présenter sous forme de solutions, émulsions, suspensions, crèmes, lotions, pommades, aérosols, poudres, de solutions ou suspensions injectables, comprimés, granulés, gélules, capsules et être administrées selon le type de formulation par voie topique ou par voie générale.

Lorsque les composés selon l'invention sont utilisés en cosmétique ou en pharmacie, la concentration en

composé actif est généralement comprise entre 0,1 et 10% en poids par rapport au poids total de la composition.

En plus de leur utilisation un cosmétique et en pharmacie humaine et vétérinaire les composés selon l'invention trouvent une application dans d'autres domaines de l'industrie tels que l'agriculture, la papeterie, les peintures et vernis, le traitement des eaux, et les produits d'entretien ménager notamment dans les produits de lavage et de désinfection du linge.

Lorsque les composés selon l'invention sont utilisés comme agents anti-algues, antifongiques et antibactériens dans les produits de traitement des eaux, par exemple dans les piscines ou dans les systèmes à refroidissement par eau, la concentration requise est généralement comprise entre 0,5 et 1.000ppm.

Lorsqu'ils sont utilisés dans des détergents en poudre ou sous forme liquide pour le linge, la concentration est de préférence comprise entre 0,01 et 10%.

Dans les peintures et vernis antifouling et antifongiques la concentration en composé actif varie de préférence entre 0,1 et 25g par litre.

On va maintenant donner à titre d'illustration et sans aucun caractère limitatif, plusieurs exemples de préparation des adamantyl-2 isothiazoline-4 ones-3 selon l'invention ainsi que certains exemples d'utilisation.

EXEMPLE 1

Préparation de l'(adamantyl-1')-2 triméthylène-4,5 isothiazoline-4 one-3

a) Préparation du benzylthio-2 cyclopentène N-(adamantyl-1') carboxamide.

A une suspension agitée sous atmosphère inerte, à température ambiante, de 50g d'acide benzylthio-2 cyclopentène carboxylique dans 300cm3 de dichlorométhane on ajoute, goutte à goutte 13cm3 de trichlorure de phosphore.

Ensuite le mélange est porté pendant trois heures au reflux du solvant. A ce stade la solution est homogène. Elle est utilisée brute pour la transformation en amide.

A cette solution refroidie à 0°C on ajoute lentement une solution contenant un mélange de 38,65g d'adamantanamine-1 (ou anantadine) et de 75cm3 de triéthylamine dans 200cm3 de chlorure de méthylène anhydre. L'addition terminée le mélange réactionnel est encore agité pendant 2 heures à température ambiante, puis il est versé dans 1,5 litre d'eau glacée. La phase organique est décantée, lavée avec une solution aqueuse d'acide chlorhydrique 1N, puis deux fois à l'eau et enfin séchée sur sulfate de magnésium. Le solvant est éliminé par évaporation sous vide et le liquide visqueux obtenu est versé dans 300cm3 d'éther isopropylique agité à 0°C. Le précipité est essoré et séché, puis recristallisé dans 400cm3 d'éther isopropylique. On obtient 54,7g de benzylthio-2 cyclopentène N-(adamantyl-1') carboxamide sous forme de cristaux blancs de point de fusion: 91°C.

Analyse élémentaire: $C_{29}H_{29}NOS$

| | | | | | |
|---|---|---|---|---|---|
| Calc: | C: 75,16 | H: 7,95 | N: 3,71 | O: 4,35 | S: 8,72 |
| Tr: | 74,60 | 8,04 | 3,79 | 4,92 | 8,62 |

b) Préparation du benzylsulfinyl-2 cyclopentène N-(adamantyl-1') carboxamide.

A une suspension agitée à 0°C de 50g de benzylthio-2 cyclopentène N-(adamantyl-1') carboxamide dans 300cm3 d'acide formique, on ajoute, goutte à goutte, 14cm3 d'eau oxygénée (30vol.). Après la fin de l'addition le milieu réactionnel est encore agité 2 heures é température ambiante puis versé directement dans un litre d'eau glacée. Le sulfoxyde est extrait au chlorure de méthylène et la phase organique est lavée avec une solution à 5% de bicarbonate de sodium puis à l'eau et enfin séchée sur sulfate de magnésium.

Après évaporation du solvant, on obtient 49g du sulfoxyde qui sont lavés avec de l'éther isopropylique. On isole 48g de benzylsulfinyl-2 cyclopentène N-(adamantyl-1') carboxamide de point de fusion: 148°C.

Analyse élémentaire: $C_{23}H_{29}NO_2S$

| | | | | | |
|---|---|---|---|---|---|
| Calc: | C: 72,02 | H: 7,62 | N: 3,65 | O: 8,34 | S: 8,36 |
| Tr: | 72,00 | 7,64 | 3,66 | 8,45 | 8,21 |

c) Préparation du chlorhydrate d'(adamantyl-1')-2 triméthylène-4,5 isothiazoline-4 one-3.

A une solution agitée à 10°C de 48g de sulfoxyde (obtenu à l'étape b) dans 150cm3 de chlorure de méthylène anhydre on ajoute, goutte à goutte 11cm3 de chlorure de thionyle. Ensuite on maintient l'agitation pendant une heure à température ambiante, puis sous forte agitation on ajoute rapidement 200cm3 d'ether isopropylique. Le précipité est essoré, séché, puis de nouveau agité dans 200cm3 d'éther isopropylique. On élimine ainsi une trace d'imputeté. Les cristaux sont essorés et séchés et on obtient 27g de cristaux blancs de point de fusion: 182°C.

Analyse élémentaire: $C_{16}H_{22}ClNOS$

| | | | | | |
|---|---|---|---|---|---|
| Calc: | C: 61,61 | H: 7,11 | N: 4,49 | Cl: 11,37 | S: 10,25 |
| Tr: | 60,98 | 7,15 | 4,36 | 11,19 | 10,12 |

d) Préparation de l'(adamantyl-1')-2 triméthylène-4,5 isothiazoline-4 one-3.

Une solution de 20g de chlorhydrate d'(adamantyl-1')-2 triméthylène-4,5 isothiazoline-4 one-3 dans 200cm3 de chlorure de méthylène est lavée trois fois à l'eau. La phase de chlorure de méthylène est décantée, séchée sur sulfate de magnésium, concentrée puis versée directement dans 500cm3 d'éther isopropylique agité à 0°C. Le solide est essoré puis séché. On obtient 11,5g d'(adamantyl-1')-2 triméthylène-4,5 isothiazoline-4 one-3 sous forme de cristaux beiges de point de fusion: 188°C.

Analyse élémentaire: $C_{16}H_{21}NOS$

| | | | | |
|---|---|---|---|---|
| Calc: | C: 69,77 | H: 7,69 | N: 5,09 | S: 11,64 |
| Tr: | 69,54 | 7,77 | 4,94 | 11,30 |

EXEMPLE II

Préparation de l'(adamantyl-1' méthyl)-2 triméthylène-4,5 isothiazoline-4 one-3.

a) Préparation du benzylthio-2 cyclopentène N-(adamantyl-1' méthyl) carboxamide.

A une suspension agitée sous atmosphère inerte de 4,7g d'acide benzylthio-2 cyclopentène carboxylique on ajoute 1,3cm3 de trichlorure de phosphore. Ce mélange est ensuite porté sous reflux pendant 3 heures, temps au bout duquel le milieu est homogène. La solution est alors refroidie à 0° C et on ajoute goutte à goutte une solution de 4g d'adamantane méthyl-1 amine et de 8,5cm3 de triéthylamine dans 25cm3 de chlorure de méthylène.

Le mélange ensuite agité 1 heure à température ambiante puis versé dans 150cm3 d'eau. La phase organique est décantée, lavée avec une solution aqueuse d'acide chlorhydrique 2N, puis 2 fois à l'eau et séchée sur sulfate de magnésium. Le chlorure de méthylène est éliminé par évaporation sous vide et le produit obtenu est agité dans 40cm3 d'éther isopropylique. Les cristaux sont essorés et séchés. On obtient 5,18g de cristaux beiges de point de fusion: 121°C.

b) Préparation du benzylsulfinyl-2 cyclopentène N-(adamantyl-1' méthyl) carboxamide.

A une solution de 48g de benzylthio-2 cyclopentène N-(adamantyl-1' méthyl) carboxamide dans 100cm3 de chlorure de méthylène agitée à 0° sous atmosphère inerte à l'abri de la lumière on ajoute par petites portions 2,3g d'acide métachloperbenzoïque. Ensuite, le mélange est agité pendant 2 heures à température ambiante, lavé avec une solution saturée de bicarbonate de sodium puis à l'eau, séchée sur sulfate de magnésium et concentrée.

La solution concentrée obtenue est versée dans 50cm3 d'éther isopropylique agité à 0°C. Le précipité obtenu est essoré puis séché et on obtient 2,85g de benzylsulfinyl-2 cyclopentène N-(adamantyl-1' méthyl) carboxamide sous forme de cristaux beiges de point de fusion: 168°C.

Ce produit est analysé sous forme hydratée.

Analyse élémentaire: $C_{24}H_{31}NO_2S$, 1/2 $H_2O$

| | | | | |
|---|---|---|---|---|
| Calc: | C: 70,89 | H: 7,93 | N: 3,44 | S: 7,88 |
| Tr: | 70,69 | 7,92 | 3,44 | 7,62 |

c) Préparation du chlorhydrate de l'(adamantyl-1' méthyl)-2 triméthylène-4,5 isothiazoline-4 one-3.

A une suspension agitée sous atmosphère inerte à 0°C de 2,6g de benzylsulfinyl-2 cyclopentène N-(adamantyl-1' méthyl) carboxamide dans 25cm3 de chlorure de méthylène on ajoute goutte à goutte 0,6cm3 de chlorure de thionyle. Le mélange est ensuite agité 1 heure à température ambiante puis versé dans 200cm3 d'éther isopropylique agité à -10°C.

Le produit précipité est essoré, dissous dans le minimum de chlorure de méthylène et précipité par addition de pentane. Les cristaux sont essorés et séchés. On obtient 1,60g de chlorhydrate d'(adamantyl-1' méthyl)-2 triméthylène-4,5 isothiazoline-4 one-3 sous forme de cristaux blancs de point de fusion: 161°C.

Analyse élémentaire: $C_{17}H_{24}ClNOS$

| | | | | | |
|---|---|---|---|---|---|
| Calc: | C: 62,65 | H: 7,42 | Cl: 10,88 | N: 4,30 | S: 9,84 |
| Tr: | 62,68 | 7,78 | 10,71 | 4,40 | 9,66 |

d) Préparation de l'(adamantyl-1′ méthyl)-2 triméthylène-4,5 isothiazoline-4 one-3.

Une solution de 1,50g du chlorure (préparé selon l'étape c) dans 100cm3 de chlorure de méthylène est lavée trois fois avec 50cm3 d'eau. Le chlorhydrate est totalement déplacé. La phase de chlorure de méthylène est décantée, séchée sur sulfate de sodium et concentrée.

Cette solution concentrée est versée dans de l'hexane glacé. Le produit cristallisé est essoré et séché. On obtient 1,20g d'(adamantyl-1′ méthyl)-2 triméthylène-4,5 isothiazoline-4 one-3 sous forme de cristaux blancs de point de fusion: 145°C.

Analyse élémentaire: $C_{17}H_{23}NOS$

| | | | | | |
|---|---|---|---|---|---|
| Calc: | C: 70,54 | H: 8,01 | N: 4,84 | O: 5,53 | S: 11,08 |
| | 70,52 | 7,79 | 4,82 | 5,70 | 10,95 |

EXEMPLE III

Préparation de l'(adamantyl-2′)-2 triméthylène-4,5 isothiazoline-4 one-3

a) Préparation du benzylthio-2 cyclopentène N-(adamantyl-2′) carboxamide.

A une solution agitée à température ambiante sous atmosphère inerte de 25,2g d'acide benzylthio-2 cyclopentène carboxylique dans 200cm3 de dichlorométhane on ajoute, goutte à goutte, 9,3cm3 de trichlorure de phosphore. L'addition n'est pas exothermique et le mélange est ensuite porté sous reflux pendant 2 heures puis abandonné à température ordinaire pendant la nuit.

Le lendemain cette solution de chlorure d'acide brut est directement ajoutée, goutte à goutte, à un mélange de 19,5g d'adamantanamine-2 et de 45, 1cm3 de triéthylamine dans 200cm3 de chlorure de méthylène anhydre refroidi à 0°C.

Le réaction est fortement exothermique. L'addition terminée, le mélange est agité 2 heures à température ambiante puis versé dans 50cm3 d'eau. La phase organique est décantée, lavée avec une solution aqueuse d'acide chlorhydrique 2N puis à l'eau jusqu'à pH neutre des eaux de lavage et enfin séchée sur sulfate de magnésium. Après évaporation sous vide du chlorure de méthylène, on obtient 28,8 de benzylthio-2 cyclopentène N-(adamantyl-2′) carboxamide brut que l'on utilise tel quel pour l'étape suivante.

b) Préparation du benzylsulfinyl-2 cyclopentène N-(adamantyl-2′) carboxamide.

A une solution agitée sous atmosphère inerte à 0°C de 29g de benzylthio-2 cyclopentène N-(adamantyl-2′) carboxamide dans 20cm3 d'acide formique, on ajoute goutte à goutte 8,1cm3 d'eau oxygénée à 110 Vol. L'addition terminée le milieu réactionnel est encore agité 2 heures à température ambiante, puis concentré sous pression réduite. Le produit obtenu est solubilisé dans 300cm3 de chlorure de méthylène, puis la solution organique est lavée avec une solution aqueuse de soude 2N, puis à l'eau jusqu'à obtention d'un pH neutre des eaux de lavage et enfin séchée sur sulfate de magnésium.

Après évaporation du chlorure de méthylène, les cristaux obtenus sont agités dans un mélange pentane-éther isopropylique puis essorés et séchés.

Les 31g de produit brut obtenu sont alors solubilisés dans le minimum de chlorure de méthylène et la solution est déposée sur une colonne de chromatographie de gel de silice que l'on élue au chlorure de méthylène, puis au mélange chlorure de méthylène-acétate d'ethyle progressivement enrichi en acétate d'éthyle. Après évaporation du solvant des fractions contenant le produit pur, on obtient 15g de benzylsulfinyl-2 cyclopentène N-(adamantyl-2′) carboxamide sous forme de cristaux blancs de point de fusion: 185°C.

Analyse élémentaire: $C_{23}H_{29}NO_2S$

| | | | | | |
|---|---|---|---|---|---|
| Calc: | C: 72,02 | H: 7,62 | N: 3,65 | O: 8,34 | S: 8,36 |
| Tr: | 71,81 | 7,69 | 3,68 | 8,09 | 8,24 |

c) Préparation du chlorhydrate d'(adamantyl-2′)-2 triméthylène-4,5 isothiazoline-4 one-3.

A une solution agitée sous atmosphère inerte à 0°C de 13g de benzylsulfinyl-2 cyclopentène N-(adamantyl-2′) carboxamide dans 100cm3 de dichlorométhane on ajoute, goutte à goutte, 13g de chlorure de thionyle. Le réaction est légèrement exothermique. L'addition terminée, le mélange est encore agité une heure à température ambiante.

Il est ensuite versé dans 200cm3 d'éther isopropylique agité à 0°C. Le précipité est essoré et séché. On obtient 8g de chlorhydrate d'(adamantyl-2')-2 triméthylène-4,5 isothiazoline-4 one-3 sous forme de cristaux beiges de point de fusion: 175°C.

Analyse élémentaire: $C_{16}H_{21}ClNOS$

| | | | | | |
|---|---|---|---|---|---|
| Calc: | C: 61,61 | H: 7,11 | Cl: 11,37 | H: 4,49 | S: 10,25 |
| Tr: | 61,39 | 7,40 | 11,26 | 4,42 | 10,18 |

d) Préparation de l'(adamantyl-2')-2 triméthylène-4,5 isothiazoline-4 one-3.

Une solution de 7,5g de chlorhydrate d'(adamantyl-2')-2 triméthylène-4,5 isothiazoline-4 one-3 dans 150cm3 de chlorure de méthylène est lavée trois fois à l'eau. Au troisième lavage, le chlorhydrate est entièrement déplacé. La phase de chlorure de méthylène est séchée sur sulfate de magnésium, puis concentrée. A cette solution concentrée, agitée à 0°C on ajoute du pentane. Le précipité est essoré et séché. On obtient 5,2g d'(adamantyl-2')-2 triméthylène-4,5 isothiazoline-4 one-3 sous forme de cristaux beiges de point de fusion: 140°C.

Analyse élémentaire: $C_{16}H_{21}NOS$

| | | | | | |
|---|---|---|---|---|---|
| Calc: | C: 69,77 | H: 7,69 | N: 5,09 | O: 5,81 | S: 11,64 |
| Tr: | 69,64 | 7,72 | 5,13 | 6,07 | 11,61 |

EXEMPLE IV

Préparation de l'(adamantyl-2')-2 isothiazolo-(5,4b) pyridinone-3.

a) Préparation du N-(adamantyl-2') chloro-2 nicotinamide

(i) Préparation du chlorure d'acide de l'acide chloro-2 nicotinique.

Une suspension de 20g d'acide chloro-2 nicotinique dans 100cm3 de chlorure de thionyle est portée sous agitation au reflux pendant deux heures. A ce stade le milieu réactionnel est homogène. Le chlorure de thionyle n'ayant pas réagi est évaporé sous pression réduite et on obtient le chlorure d'acide sous forme de paillettes nacrées blanches que l'on solubilise dans 50cm3 de dichlorométhane anhydre.

(ii) Préparation de l'amide.

Cette solution du chlorure d'acide est ajoutée goutte à goutte à un mélange fortement agité sous atmosphère inerte à 0°C de 23g d'adamantanamine-2 et de 89cm3 de triéthylamine dans 250cm3 de chlorure de méthylène anhydre. L'addition de chlorure d'acide terminée, on agite le mélange hétérogène obtenu, encore 2 heures à température ambiante. Il est ensuite versé dans 400cm3 d'eau et la phase organique est décantée, lavée avec une solution d'acide chlorhydrique normale puis à l'eau. La phase organique est séchée sur sulfate de magnésium puis concentrée sous pression réduite. Le solide beige obtenu est solubilisé dans 100cm3 de chlorure de méthylène anhydre. Après refroidissement les cristaux obtenus sont essorés et séchés. On obtient 25g de N-(adamantyl-2') chloro-2 nicotinamide dont le spectre de $^1H$ R.M.N correspond à la structure attendue.

b) Préparation du N-(adamantyl-2') benzylthio-2 nicotinamide.

On prépare dans un premier stade le benzylthiolate de potassium en solubilisant 12,3cm3 de benzylmercaptan dans 300cm3 d'éthoxy-2 éthanol auquel on ajoute 6,9g de potasse en pastilles. A cette solution agitée sous atmosphère inerte à température ambiante on ajoute par portions les 25g de N-(adamantyl-2') chloro-2 nicotinamide. Le mélange est encore agité trois heures, un insoluble apparaît progressivement.

Le lendemain ce dernier est essoré, puis solubilisé dans 300cm3 de dichlorométhane. La solution obtenue est lavée trois fois avec 200cm3 d'eau et la phase organique est séchée sur sulfate de magnésium.

Le solvant est éliminé par évaporation sous vide et le solide obtenu est agité dans 100cm3 d'ether isopropylique dans le but de bien le diviser.

Il est essoré et séché. On obtient 26g de N-(adamantyl-2') benzylthio-2 nicotinamide sous forme de paillettes nacrées blanches de point de fusion: 170°C.

Analyse élémentaire: $C_{23}H_{26}N_2OS$

| | | | | | |
|---|---|---|---|---|---|
| Calc: | C: 72,98 | H: 6,92 | N: 7,40 | O: 4,23 | S: 8,47 |
| Tr: | 72,77 | 7,01 | 7,28 | 4,38 | 8,28 |

c) Préparation du N-(adamantyl-2′) benzylsulfinyl-2 nicotinamide.

A une solution agitée sous atmosphère inerte à 0° C de 25g de N-(adamantyl-2′) benzylthio-2 nicotinamide dans 200cm3 d'acide formique on ajoute 7,5cm3 d'eau oxygénée (110 Vol).

La solution est ensuite agitée pendant 3 heures à température ambiante.

L'acide formique est éliminé par évaporation sous vide et le produit obtenu est solubilisé dans 300cm3 de dichlorométhane. Cette solution est lavée avec une solution normale de soude puis à l'eau.

La phase organique est décantée, séchée sur sulfate de magnésium et le solvant est éliminé par évaporation sous pression réduite. On obtient un solide qui est lavé à l'éther isopropylique et séché. Les 22g de N-(adamantyl-2′) benzylsulfinyl-2 nicotinamide ainsi isolés ont un point de fusion de: 218° C.

Analyse élémentaire: $C_{23}H_{26}N_2O_2S$

| | | | | | |
|---|---|---|---|---|---|
| Calc: | C: 70,02 | H: 6,64 | N: 7,10 | O: 8,11 | S: 8,13 |
| Tr: | 69,97 | 6,66 | 6,97 | 8,21 | 7,94 |

d) Préparation du chlorhydrate d'(adamantyl-2′)-2 isothiazolo- (5,4b) pyridinone-3.

A une solution agitée à 0° C sous atmosphère inerte de 21g de N-(adamantyl-2′) benzylsulfinyl-2 nicotinamide dans 100cm3 de chlorure de thionyle. Un produit précipite et le mélange est encore agité pendant 1 heure à température ambiante. Le précipité est essoré, lavé à l'éther isopropylique puis au pentane et enfin séché.

On obtient 15g de chlorhydrate d'(adamantyl-2′)-2 isothiazolo- (5,4b) pyridinone-3 sous forme de cristaux jaunes de point de fusion: 188° C.

Analyse élémentaire: $C_{16}H_{19}Cl\ N_2OS$

| | | | | | | |
|---|---|---|---|---|---|---|
| Calc: | C: 59,52 | H: 5,93 | Cl: 10,98 | N: 8,68 | O: 4,96 | S: 9,93 |
| Tr: | 59,53 | 5,94 | 10,89 | 8,75 | 5,22 | 9,86 |

e) Préparation de l'(adamantyl-2′)-2 isothiazolo-(5,4b) pyridinone-3.

Une solution de 13g du chlorhydrate précédent dans 500cm3 de chlorure de méthylène est agitée en présence de 200cm3 d'une solution aqueuse de soude.

La phase organique est décantée, lavée encore trois fois avec de l'eau pure puis séchée sur sulfate de magnésium. Le chlorure de méthylène est ensuite éliminé par évaporation sous vide et le solide obtenu est alors fortement agité dans 100cm3 de pentane, puis essoré et séché. On obtient 10,5g d'(adamantyl-2′)-2 isothiazolo-(5,4b) pyridinone-3 sous forme de cristaux blancs de point de fusion: 108° C.

Analyse élémentaire: $C_{16}H_{18}N_2OS$

| | | | | | |
|---|---|---|---|---|---|
| Calc: | C: 67,10 | H: 6,33 | N: 9,78 | O: 5,59 | S: 11,20 |
| Tr: | 66,89 | 6,32 | 9,88 | 5,75 | 11,07 |

<u>Préparation des intermédiaires de synthèse de l'(adamantyl-2′)-2 isothiazoline-4 one-3 et de ses homologues halogénés (chlorés) dans les positions 4 ou 5 et 5.</u>

<u>A. Préparation du N-(adamantyl-2′) chloro-3 propionamide.</u>

A une solution agitée à 0° C sous atmosphère inerte de 106g de chlorure de l'acide chloro-3 propionique dans 400cm3 de dichloro-1,2 éthane on introduit, goutte à goutte, un mélange contenant 120g d'adamantanamine-2 et 80,32g de triéthylamine dissous dans 1 litre de dichloro-1,2 éthane.

La réaction est exothermique et la vitesse d'introduction est réglée de façon à ce que la température ne dépasse pas 5° C. Après la fin de l'introduction le mélange est encore agité pendant 1h30mn.

A ce stade la majorité du produit de départ est transformée, on introduit alors 500cm3 d'acide chlorhydrique 3N sous agitation. La phase organique est ensuite décantée, lavée trois fois à l'eau, séchée sur sulfate de sodium et concentrée. Le produit brut obtenu est recristallisé dans un mélange de 1 litre d'éther isopropylique et 350cm3 d'acétate d'éthyle.

On obtient 150g de N-(adamantyl-2′) chloro-3 propionamide sous forme de cristaux blancs de point de fusion: 125-126° C.

Le spectre $^1H$ R.M.N. est conforme à la structure attendue.

B. Préparation de l'acétylthio-3 N-(adamantyl-2') propionamide.

A une solution agitée à la température ordinaire, sous atmosphère inerte de 147g de N-(adamantyl-2') chloro-3 propionamide dans 1,5 litre d'acétone anhydre on ajoute par petites quantités 69,32g de thiolacétate de potassium. Le mélange est ensuite porté progressivement sous reflux du solvant. Quatre heures plus tard la totalité du produit de départ est transformée .

L'acétone est alors évaporée sous pression réduite et le solide obtenu est dissous dans du dichlorométhane. La solution obtenue est lavée trois fois à l'eau puis séchée sur sulfate de sodium et concentrée.

On obtient 165g de solide que l'on dissout dans un mélange éther isopropylique-toluène (1-1) bouillant en présence de noir animal.

La solution bouillante est filtrée puis refroidie. L'acétyl-thio-3 N-(adamantyl-2') propionamide cristallisée est est essorée puis séchée . On isole 140g de produit sous forme de cristaux beiges de point de fusion : 124° C.

Analyse élémentaire: $C_{15}H_{23}NO_2S$

| | | | | | |
|---|---|---|---|---|---|
| Calc: | C: 64,02 | H: 8,23 | N: 4,97 | O: 11,37 | S: 11,39 |
| Tr: | 64,47 | 8,29 | 4,89 | 11,58 | 11,09 |

C. Préparation du N-(adamantyl-2') mercapto-3 propionamide.

Une solution agitée sous atmosphère inerte, de 46g d'acétylthio-3 N-(adamantyl-2') propionamide dans un mélange de 1 litre de méthanol et 50cm3 d'acide chlorhydrique 2 N est portée au reflux du solvant pendant 6 heures. Le solvant est alors éliminé sous pression réduite et le solide obtenu est dissous dans du chlorure de méthylène.

La solution est ensuite lavée plusieurs fois à l'eau, séchée sur sulfate de sodium puis concentrée. On obtient 43g de N-(adamantyl-2') mercapto-3 propionamide sous forme d'une poudre blanche dont le spectre $^1H$ R.M.N est conforme à la structure.

D. Préparation du disulfure de N-(adamantyl-2') mercapto-3 propionamide.

Une solution de 25,92g de N-(adamantyl-2') mercapto-3 propionamide dans 500cm3 de méthanol est agitée à une température comprise entre 0° et 5° C. On y ajoute goutte à goutte 20cm3 d'ammoniaque concentré puis 10cm3 d'eau oxygénée (110Vol.). Le mélange est ensuite agité à la température ordinaire jusqu'à ce que la totalité du produit de départ soit transformée. Le disulfure cristallise au sein du mélange réactionnel et après refroidissement, le solide est essoré puis séché et recristallisé dans le méthanol. On obtient 30g du disulfure du N-(adamantyl-2') mercapto-3 propionamide sous forme de cristaux blancs de point de fusion: 199° C.

Analyse: $C_{26}H_{40}N_2O_2S_2$

| | | | | | |
|---|---|---|---|---|---|
| Calc: | C: 65,50 | H: 8,45 | N: 5,87 | O: 6,71 | S: 13,45 |
| Tr: | 65,38 | 8,41 | 5,90 | 6,86 | 13,45 |

E. Préparation du N-(adamantyl-1')chloro-3 propionamide

A une solution agitée à une température d'environ - 10° C de 29g de chlorure de l'acide chloro-3 propionique dans 400 $cm^3$ de dichloro-1,2 éthane, on introduit, goutte à goutte, une solution contenant 68,5g d'adamantamine-1 (2 équivalents) dans 600 $cm^3$ de dichloro-1,2 éthane. La réaction est exothermique et la vitesse d'introduction est réglée de façon à maintenir la température entre - 8° C et - 12° C.

A la fin l'introduction, on laisse la température revenir vers 0° C, température à laquelle, le précipité blanc de chlorhydrate d'adamantamine-1 est essoré. Le filtrat est lavé à l'acide chlorhydrique 0,1 N, puis à l'eau et séché sur sulfate de sodium. Après élimination du solvant, l'amide brut est recristallisé dans l'éther isopropylique. On obtient 45g de N-(adamantyl-1') chloro-3 propionamide sous forme de cristaux blancs de point de fusion : 121° C.

Analyse élémentaire : $C_{13}H_{20}ClNO$

| | | | | | |
|---|---|---|---|---|---|
| Calc: | C: 64,58 | H: 8,34 | Cl: 14,67 | N: 5,79 | O: 6,62 |
| Tr | 64,18 | 8,40 | 15,23 | 5,82 | 6,66 |

F. Préparation de l'acétylthio-3 N-(adamantyl-1') propionamide

A une solution agitée à la température ambiante, sous atmosphère inerte, de 42,4g de N-(adamantyl-1') chloro-3 propionamide dans 600 $cm^3$ d'acétone anhydre, on ajoute par petites quantités 20g de thiolacétate de potassium. Le mélange est ensuite porté progressivement au reflux du solvant. Six heures plus tard la majorité du produit de départ est transformé et l'acétone est alors évaporée. Le produit est ensuite dissous dans un mélange de chlorure de méthylène et d'eau et la phase organique est décantée, lavée trois fois à l'eau,

séchée sur sulfate de sodium puis concentrée.

Après recristallisation du produit brut obtenu dans l'éther isopropylique on obteint 42g d'acétylthio-3 N-(adamantyl-1') propionamide sous forme de cristaux blancs de point de fusion : 104°C.

Analyse élémentaire : $C_{15}H_{23}NO_2S$

| | | | | | |
|---|---|---|---|---|---|
| Calc | C: 64,02 | H: 8,24 | N: 4,98 | O: 11,37 | S: 11,39 |
| Tr | 63,73 | 8,24 | 5,09 | 11,56 | 11,63 |

EXEMPLE V

Préparation de l'(adamantyl-2')-2 isothiazoline-4 one-3.

On prépare une solution de 18,4g de chlore dans 800cm3 d'acétate d'éthyle anhydre. On ajoute, sous agitation, à cette solution, à la température ordinaire, 40g d'acétylthio-3 N-(adamantyl-2') propionamide sous forme solide. La température s'élève à 34°C, le milieu devient homogène, puis une demi-heure plus tard un insoluble se forme.

L'agitation est maintenue trois heures supplémentaires puis le précipité est essoré et séché. On obtient 15,20g d'(adamantyl-2')-2 isothiazoline-4 one-3 brute sous forme de chlorhydrate que l'on dissout dans 300cm3 de chlorure de méthylène. La solution obtenue est lavée trois fois à l'eau, séchée sur sulfate de sodium et évaporée sous pression réduite.

Le solide obtenu contenant des impuretés, est extrait plusieurs fois à l'hexane bouillant et les solutions d'hexane sont alors concentrées jusqu'au moment où l'on observe un début de cristallisation.

Le mélange est alors refroidi et les cristaux sont essorés, puis séchés. On obtient 10g d'(adamantyl-2')-2 isothiazoline-4 one-3 sous forme de cristaux blancs de point de fusion: 153°C.

Analyse élémentaire: $C_{13}H_{17}NOS$

| | | | | | |
|---|---|---|---|---|---|
| Calc: | C: 66,34 | H: 7,28 | N: 5,95 | O: 6,80 | S: 13,62 |
| Tr: | 66,40 | 7,26 | 5,93 | 7,03 | 13,54 |

EXEMPLE VI

Préparation de l'(adamantyl-2')-2 chloro-5 isothiazoline-4 one-3.

Une solution agitée à l'abri de la lumière, de 87g de chlorure de sulfuryle, dans 1,4 litre de toluène anhydre est refroidie à une température comprise entre -15° et 10°C. A celle-ci on ajoute rapidement 60g d'acétylthio-3 N-(adamantyl-2') propionamide sous forme solide. Pendant quelques minutes le mélange réactionnel est homogène puis rapidement apparaît un insoluble. Une demi-heure après la fin de l'addition on laisse le mélange revenir à la température ambiante (20°C) à ce stade le mélange est homogène. L'agitation est encore maintenue pendant 3 heures à température ordinaire, puis on ajoute 1 litre d'eau et ensuite du bicarbonate de sodium pour amener le pH de la phase aqueuse à environ 5,5. La phase organique est alors décantée, lavée à l'eau puis séchée sur sulfate de sodium et enfin concentrée sous pression réduite. On obtient 62,5g d'un produit brut sous forme d'un liquide visqueux que l'on chromatographie par passage sur colonne de chromatographie de gel de silice. Le produit attendu est élué au chlorure de méthylène puis au mélange chlorure de méthylène-acétate d'éthyle. Après élimination du solvant sous pression réduite on obtient 40g d'(adamantyl-2')-2 chloro-5 isothiazoline-4 one-3.

Après recristallisation dans l'acétate d'éthyle on isole 25g de cristaux blancs de point de fusion: 111°C.

Analyse élémentaire: $C_{13}H_{16}Cl$ NOS

| | | | | | | |
|---|---|---|---|---|---|---|
| Calc: | C: 57,87 | H: 5,97 | Cl: 13,14 | N: 5,19 | O: 5,93 | S: 11,88 |
| Tr: | 57,97 | 5,97 | 13,30 | 5,17 | 6,07 | 11,77 |

EXEMPLE VII

Préparation de l'(adamantyl-2')-2 dichloro-4,5 isothiazoline-4 one-3.

Dans une solution de 15g de disulfure du N-(adamantyl-2') mercapto-3 propionamide dans 500cm3 de dichloro-1,2 éthane on fait passer lentement un courant de chlore de façon à en capter environ 23g pendant environ 3 heures. La température de la solution agitée est maintenue pendant ce temps à 20°C.

Ensuite on fait passer un fort courant d'azote gazeux dans la solution pour éliminer le chlore n'ayant pas réagi ainsi que le gaz chlorhydrique. On ajoute alors environ 300cm3 d'eau sous agitation et le pH de la phase aqueuse est amené à environ 5,5 par addition de bicarbonate de sodium. La phase organique est décantée, lavée à l'eau, séchée sur sulfate de sodium et concentrée.

Cette solution concentrée est déposée sur une colonne de chromatographie de gel de silice et celle-ci est éluée au chlorure de méthylène puis au mélange chlorure de méthylène-éthanol de façon à obtenir des fractions enrichies en dérivé dichloré. Ces différentes fractions enrichies sont évaporées sous vide, puis le produit obtenu solubilisé dans du toluène est déposé à nouveau sur une colonne de chromatographie de gel de silice cette fois éluée au toluène progressivement enrichi au chlorure de méthylène et ensuite au mélange chlorure de méthylène-acétate d'éthyle. Les fractions contenant le dérivé dichloré sont rassemblées puis évaporées sous vide. Le solide obtenu est recristallisé dans le minimum d'acétate d'éthyle. On obtient 3g d'(adamantyl-2')-2 dichloro-4,5 isothiazoline-4 one-3 sous forme d'aiguilles beiges de point de fusion: 142°C.

Analyse élémentaire: $C_{13}H_{15}Cl_2NOS$

| | | | | | |
|---|---|---|---|---|---|
| Calc: | C: 51,32 | H: 4,96 | N: 4,60 | O: 5,25 | S: 10,53 |
| Tr: | 51,38 | 4,97 | 4,72 | 5,45 | 10,40 |

EXEMPLE VIII

Préparation de l'(adamantyl-1')-2 isothiazoline-4 one-3

A une solution agitée à la température ambiante de 10g d'acétylthio-3 N-(adamantyl-1') propionamide dans 250 $cm^3$ de chlorure de méthylène anhydre, on ajoute goutte à goutte 7,2g de chlorure de sulfuryle (1,5 équivalent) dilué dans 60 $cm^3$ de chlorure de méthylène. Deux heures après la fin de l'introduction, on ajoute lentement sous agitation, en maintenant la température inférieure à 20°C, une solution de bicarbonate de sodium jusqu'à ce que le pH de la phase aqueuse soit d'environ 6. La phase organique est ensuite décantée, lavée à l'eau, séchée sur sulfate de sodium puis concentrée et enfin déposée sur une colonne de chromatographie
gel de silice que l'on élue au chlorure de méthylène.

Après évaporation des solutions contenant le produit attendu et recristallisation dans l'acétone, on obtient 4g d'(adamantyl-1')-2 isothiazoline-4 one-3 sous forme de cristaux blancs de point de fusion : 169°C.

Analyse élémentaire $C_{13}H_{17}NOS$

| | | | | | |
|---|---|---|---|---|---|
| Calc. | C: 66,34 | H: 7,28 | N: 5,95 | O: 6,80 | S: 13,62 |
| Tr. | 66,27 | 7,32 | 5,96 | 7,04 | 13,74 |

EXEMPLE IX

Préparation de l'(adamantyl-1')-2 chloro-5 isothiazoline-4 one-3 et le l'(adamantyl-1')-2 dichloro-4,5 isothiazoline-4 one-3

A une solution agitée à température ambiante et sous atmosphère inerte de 10g d'acétylthio-3 N-(adamantyl-1') propionamide dans 200 $cm^3$ de toluène anhydre on ajoute, goutte à goutte, 10 $cm^3$ de chlorure de sulfuryle (3,5 équivalents) de façon à ce que la température ne dépasse pas 24°C. Le précipité qui se forme au début de l'introduction se solubilise progressivement. Trois heures après la fin de l'introduction on ajoute 20 $cm^3$ d'eau puis, sous agitation, on introduit lentement une solution de bicarbonate de sodium de façon à amener le pH de la phase aqueuse à environ 6. La phase organique est ensuite décantée, lavée plusieurs fois à l'eau, séchée sur sulfate de sodium et le solvant est éliminé par évaporation sous vide. Les 8,5g de produit brut obtenu, solubilisé dans le minimum de chlorure de méthylène, sont déposés sur une colonne de chromatographie gel de silice et on élue au chlorure de méthylène puis au mélange chlorure de méthylène - acétate d'éthyle progressivement enrichi en acétate d'éthyle.

Après évaporation des solutions contenant les produits purifiés on isole, en tête de chromatographie, 6,2g d'(adamantyl-1')-2 chloro-5 isothiazoline-4 one-3. Après recristallisation dans le minimum d'acétate d'éthyle, on obtient 3,6g de cristaux blancs de point de fusion : 121°C.

Analyse élémentaire $C_{13}H_{16}$ Cl NOS

| | | | | | | |
|---|---|---|---|---|---|---|
| Calc. | C: 57,87 | H: 5,97 | Cl: 13,14 | N: 5,19 | O: 5,93 | S: 11,88 |
| Tr. | 57,91 | 6,00 | 13,21 | 5,09 | 6,01 | 11,67 |

Dans les mélanges plus riches en acétate d'éthyle le dérivé dichloré est entrainé et isolé après évaporation de l'éluant puis recristallisation dans l'acétate d'éthyle. On obtient 0,5g d'(adamantyl-1')-2 dichloro-4,5 isothiazoline-4 one-3 sous forme de cristaux beige de point de fusion : 202°C.

Analyse élémentaire : $C_{13}H_{15}Cl_2$ NOS

| | | | | | | |
|---|---|---|---|---|---|---|
| Calc. | C: 51,32 | H: 4,96 | Cl: 23,30 | N: 4,60 | O: 5,25 | S: 10,53 |
| Tr. | 51,43 | 4,98 | 23,08 | 4,52 | 5,40 | 10,38 |

## EXEMPLES DE FORMULATIONS

### EXEMPLE A : CREME NON IONIQUE

| | |
|---|---|
| - (adamantyl--2')-2 isothiazolo-(5,4b) pyridinone-3..... | 1,00g |
| - Monostéarate de Sorbitan polyoxyéthylèné à 20 moles d'O.E...... | 3,50g |
| - Monostéarate de Sorbitan..... | 1,50g |
| - Monostéarate de Glycérol..... | 14,00g |
| - Alcool cétylique..... | 0,50g |
| - Octyl-2 dodécanol..... | 3,00g |
| - Décyl ester de l'acide oléique..... | 6,00g |
| - Glycérol bidistillé..... | 5,00g |
| - Eau stérile déminéralisée..... | 65,50g |

### EXEMPLE B : GEL HYDROETHANOLIQUE

| | |
|---|---|
| - (adamantyl--1')-2 triméthylène-4,5 isothiazoline-4 one-3..... | 0,10g |
| - Ethanol..... | 30,00g |
| - Propylène glycol..... | 30,00g |
| - Hydroxypropylcellulose..... | 2,00g |
| - Eau déminéralisée..... | 37,90g |

EXEMPLE C : SAVON CREME

| | |
|---|---|
| - (adamantyl--2')-2 isothiazoline-4 one-3..... | 5,00g |
| - Mélange de lauryl éther sulfate de sodium et de diéthanolamide de coco à 35% de matière active..... | 87,00g |
| - Diméthylamidobétaïne de coco..... | 8,00g |

EXEMPLE D : COMPRIME DE 0,5g POUR ADMINISTRATION ORALE

| | |
|---|---|
| - (adamantyl--2')-2 isothiazoline-4 one-3..... | 0,250g |
| - Amidon..... | 0,038g |
| - Phosphate bicalcique..... | 0,100g |
| - Lactose..... | 0,075g |
| - Talc..... | 0,025g |
| - Stéarate de magnésium..... | 0,012g |

Dans cet exemple, le composé actif peut être remplacé par 1'(adamantyl-2')-2 isothiazolo-(5,4)b pyridinone-3.

EXEMPLE E : COMPRIME EFFERVESCENT DE 2g

- (adamantyl-- 2')-2 chloro-5 isothiazoline-4 one-3..... 0,250g
- Saccharose finement pulvérisé..... 1,545g
- Bicarbonate de soude..... 0,065g
- Acide citrique granulé..... 0,110g
- Gomme arabique..... 0,015g
- Acide stéarique..... 0,005g
- Arôme en poudre..... 0,010g

Dans cet exemple, le composé actif peut être remplacé par 1'(adamantyl-1')-2 triméthylène-4,5 isothiazoline-4 one-3.

EXEMPLE F : POUDRE POUR GELULE

- (adamantyl-- 2')-2 dichloro-4,5 isothiazoline-4 one-3..... 0,10g
- Amidon..... 0,06g
- Stéarate de magnésium..... 0,01g
- Saccharose..... 0,26g

Cette poudre est conditionnée dans des gélules standard.

Les composés suivants:
- (adamantyl-2')-2 isothiazoline-4 one-3,
- (adamantyl-2')-2 chloro-5 isothiazoline-4 one-3, et
- (adamantyl-2')-2 dichloro-4,5 isothiazoline-4 one-3,

se sont montrés particulièrement utiles comme agents anti-algues, antifongiques ou antibactériens dans le traitement des eaux, dans les détergents en poudre ou sous forme liquide et dans les peintures et vernis industriels.

**Revendications**

1. Adamantyl-2 isothiazoline-4 ones-3, caractérisées par le fait qu'elles répondent à la formule générale suivante:

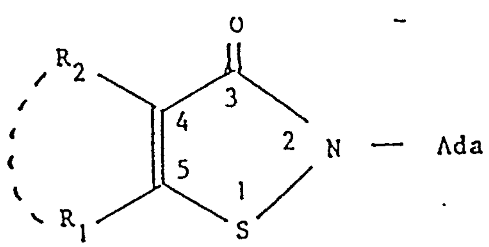

(I)

dans laquelle:

$R_1$ et $R_2$, identiques ou différents, représentent un atome d'hydrogène, ou un atome d'halogène,
ou $R_1$ et $R_2$, pris ensemble, forment une chaîne polyméthylène ayant 3 ou 4 atomes de carbone éventuellement substituée par un radical alkyle inférieur ou par un halogène,
ou encore $R_1$ et $R_2$, pris ensemble, forment un cycle pyridinique avec la double liaison du noyau isothiazoline-4 one-3,
et Ada représente un radical adamantyle pris dans le groupe constitué par:

(i) 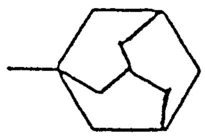 (Adamantyl-1')

(ii) 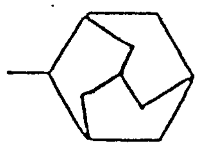 (Adamantyl-2')

(iii) 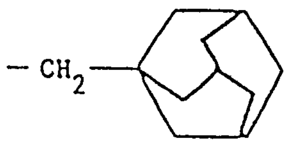 (Adamantyl-1' méthyl)

et leurs sel d'un acide minéral ou organique.

2. Composés selon la revendication 1, caractérisés par le fait que l'atome d'halogène est un atome de fluor, de brome ou de chlore.

3. Composés selon la revendication 1, caractérisés par le fait que lorsque les radicaux $R_1$ et $R_2$, pris ensemble, forment un cycle pyridinique, avec la double liaison du noyau isothiazoline-4 one-3, celui-ci est substitué par un ou plusieurs alkyle(s) inférieur(s) ayant de 1 à 4 atomes de carbone, un atome d'halogène ou un groupement nitro.

4. Composés selon la revendication 1, caractérisés par le fait qu'ils répondent à la formule générale suivante:

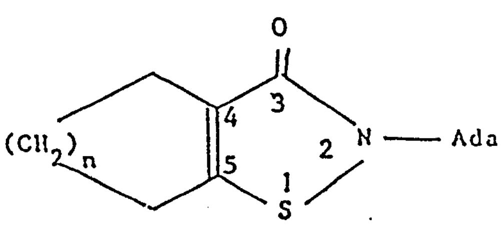 (II)

dans laquelle:
Ada à la même signification que celle donnée à la revendication 1,
et n est 1 ou 2.

5. Composés selon la revendication 1, caractérisés par le fait qu'ils répondent à la formule générale suivante:

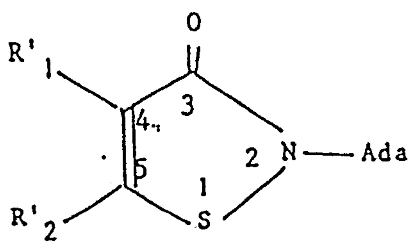 (III)

dans laquelle:
Ada a la même signification que cell donnée à la revendication 1,
et R' et $R'_2$, identiques ou différents, représentent un atome d'hydrogène ou un atome d'halogène tel qu'un atome de fluor, de chlore ou de brome.

6. Composés selon la revendication 5, caractérisés par le fait que le radical Ada représente le radical Adamantyl-1' ou Adamantyl-2' et $R'_1$ et $R'_2$ représentent un atome d'hydrogène ou un atome de chlore,

l'un au moins des radicaux $R'_1$ ou $R'_2$ représentant un atome de chlore.

7. Composés selon la revendication 1, caractérisés par le fait qu'ils répondent à la formule générale suivante:

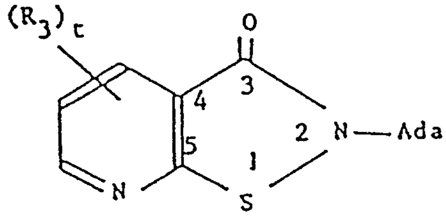

(IV)

dans laquelle:
Ada a la même signification que celle donnée à la revendication 1,
et $R_3$ représente un atome d'hydrogène, un radical alkyle inférieur ayant de 1 à 4 atomes de carbone, un atome de fluor, de chlore ou de brome ou un groupement nitro,
et t est 1 ou 2.

8. Composés selon l'une quelconque des revendications précédentes, caractérisés par le fait qu'ils se présentent sous forme de chlorhydrates, bromhydrates, nitrates, sulfates ou succinates.

9. Composés selon l'une quelconque des revendications précédentes, caractérisés par le fait qu'ils sont pris dans le groupe constitué par:
- (Adamantyl-1')-2-triméthylène-4,5 isothiazoline-4 one-3,
- (Adamantyl-1' méthyl)-2-triméthylène-4,5 isothiazoline-4 one-3,
- (Adamantyl-2')-2-triméthylène-4,5 isothiazoline-4 one-3,
- (Adamantyl-2')-2 isothiazolo-(5,4b) pyridinone-3,
- (Adamantyl-2')-2 isothiazoline-4 one-3,
- (Adamantyl-2')-2 chloro-5 isothiazoline-4 one-3,
- (Adamantyl-2')-2 dichloro-4,5 isothiazoline-4 one-3,
- (Adamantyl-1')-2 isothiazoline-4 one-3,
- (Adamantyl-1')-2 chloro-5 isothiazoline-4 one-3,
- (Adamantyl-1')-2 dichloro-4,5 isothiazoline-4 one-3,
- (Adamantyl-1')-2 bromo-5 isothiazoline-4 one-3,
- (Adamantyl-1')-2 bromo-4 chloro-5 isothiazoline-4 one-3 et
- (Adamantyl-2')-2 bromo-4 chloro-5 isothiazoline-4 one-3.

10. Procédé de préparation des composés répondant à la formule générale suivante:

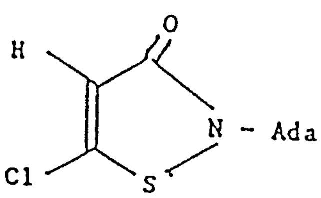

dans laquelle:
Ada a la même signification que celle donnée à la revendication 1,
caractérisé par le fait que l'on fait réagir en milieu solvant organique le chlorure de sulfuryle sur un N-adamantyl acétylthio-3 propionamide de formule: $CH_3CO-S-CH_2-CH_2-CONH$ - Ada.

11. Composés intermédiaires de synthèse, caractérisés par le fait qu'ils répondent à la formule générale suivante:

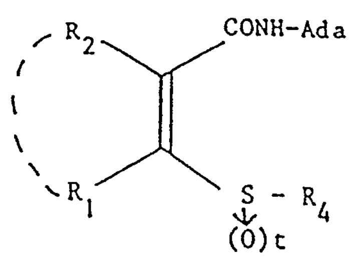

(V)

dans laquelle:
t est 0 ou 1,
Ada a la même signification que celle donnée à la revendication 1,
$R_1$ et $R_2$ ont les mêmes significations que celles données à la revendication 1 à l'exclusion de $R_1$ et $R_2$, identiques ou différents, représentant un atome d'hydrogène ou un atome d'halogène, et

$R_4$ représente un radical alkyle ou aralkyle.

12. Utilisation des composés actifs selon l'une quelconque des revendications 1 à 9 comme agents anti-bactériens, anti-algues et antifongiques dans des produits et traitements industriels.

13. Utilisation selon la revendication 12 dans le traitement des eaux à raison de 0,5 à 1000ppm de composé actif.

14. Utilisation selon la revendication 12, dans des détergents en poudre ou sous forme liquide à raison de 0,01 à 10% en poids de composé actif.

15. Utilisation selon la revendication 12, dans des peintures et vernis à raison de 0,1 à 25g de composé actif par litre.

16. Composition cosmétique, caractérisée par le fait qu'elle contient au moins un composé actif selon l'une quelconque des revendications 1 à 9.

17. Composition pharmaceutique à usage humain ou vétérinaire, caractérisée par le fait qu'elle contient au moins un composé actif selon l'un quelconque des revendications 1 à 9.

18. Composition selon l'une des revendications 16 ou 17, caractérisée par le fait qu'elle contient de 0,1 à 10% en poids dudit composé actif.

Office européen des brevets

# RAPPORT DE RECHERCHE EUROPEENNE

Numero de la demande

EP 89 40 1259

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-3 706 758 (J.C. GRIVAS) * En entier * --- | 1,12 | C 07 D 275/02<br>C 07 D 275/04<br>C 07 D 513/00<br>A 61 K 31/41<br>A 61 K 31/435<br>A 61 K 7/00<br>A 61 K 7/06<br>A 61 K 7/48<br>C 07 C 149/00<br>C 07 D 213/81<br>C 09 D 5/14<br>D 21 H 5/22<br>C 02 F 1/50 |
| A | GB-A-2 087 388 (CIRD) * Revendications * ----- | 1-18 | |
| | | | **DOMAINES TECHNIQUES RECHERCHES (Int. Cl.4)** |
| | | | C 07 D 275/00<br>C 07 D 513/00 |

Le présent rapport a été établi pour toutes les revendications

| Lieu de la recherche | Date d'achèvement de la recherche | Examinateur |
|---|---|---|
| LA HAYE | 27-07-1989 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)